Europäisches Patentamt

European Patent Office

Office européen des brevets

(11) Numéro de publication: **0 096 139**
**A1**

(12) # DEMANDE DE BREVET EUROPEEN

(21) Numéro de dépôt: **82450010.2**

(22) Date de dépôt: **10.06.82**

(51) Int. Cl.³: **A 61 L 2/06**

(43) Date de publication de la demande:
**21.12.83 Bulletin 83/51**

(84) Etats contractants désignés:
**DE FR**

(71) Demandeur: **S.A.R.L. COMMODORE INTERNATIONAL
Complexe Artisano Industriel Secteur U
F-33290 Blanquefort(FR)**

(72) Inventeur: **Baguet, Paul
10 avenue des Mésanges
F-33320 Le Taillan(FR)**

(74) Mandataire: **Thébault, Jean-Louis
Cabinet Jean-Louis Thébault 50, Cours de Verdun
F-33000 Bordeaux(FR)**

(54) Nouveau procédé de stérilisation d'objets à la vapeur et dispositifs pour sa mise en oeuvre.

(57) La présente invention concerne un procédé de stérilisation à la vapeur et de séchage d'objets solides, ainsi que les dispositifs pour sa mise en oeuvre. Ce procédé de stérilisation consiste à enfermer les objets avec une quantité d'eau déminéralisée déterminée dans un récipient, à admettre dans l'enceinte un fluide caloporteur à une température de l'ordre de 120°C à 135°C environ en vue de vaporiser l'eau à l'intérieur du récipient, à laisser l'intérieur de l'enceinte à ladite température pendant un temps suffisant pour assurer la stérilisation, puis à extraire la vapeur d'eau contenue dans ce dernier en mettant en dépression l'intérieur du récipient et, enfin, à remettre l'intérieur du récipient à la pression atmosphérique ou au voisinage de celle-ci et à retirer le récipient de ladite enceinte.
Application à la stérilisation d'objets solides.

EP 0 096 139 A1

## - 1 -
## NOUVEAU PROCÉDÉ DE STÉRILISATION D'OBJETS À LA VAPEUR ET DISPOSITIFS POUR SA MISE EN OEUVRE

La présente invention concerne un nouveau procédé de stérilisation en autoclave d'objets, permettant de réaliser une stérilisation à la vapeur et un séchage desdits objets et de conserver, même très longtemps après autoclavage, la stérilité de ces objets.

Il existe deux types de stérilisation thermique d'objets solides utilisés communément dans le domaine de la santé: la stérilisation par la température, à l'air sec, et la stérilisation par la vapeur d'eau.

La première s'opère dans des étuves dans lesquelles on introduit de l'air sec à environ 180°C, cette température étant nécessaire pour détruire les germes par éclatement des cellules. Cette technique, si elle réalise de surcroît un bon séchage des objets traités, présente néanmoins l'inconvénient majeur de fragiliser certains des objets tels que de l'outillage ou des pièces textiles du fait de l'action de la chaleur et des importantes variations de températures subies par les matières ou matériaux constitutifs de ces objets, lesquels risquent ainsi de voir leurs qualités se détériorer ou de devenir cassants et donc de voir leur durée de vie utile très sensiblement restreinte.

L'autre technique de stérilisation par la vapeur en autoclave ne nécessite qu'une température de l'ordre de 130°C pendant 15mn qui, elle, ne risque pas d'endommager les objets à traiter mais est de conduite plus délicate car il faut opérer en vapeur saturée avec moins de 1 % d'air pour qu'il ne se forme pas de poche d'air instérilisable puisque la température est très inférieure à 180°C.

Cette seconde technique est mise en oeuvre habituellement à l'aide de récipients non étanches, tels que des tambours de stérilisation, dans lesquels on enferme les objets à traiter et qui sont placés en autoclave.

Cette manière de procéder n'assure ni le séchage total des objets ni la conservation de la stérilité après sortie de l'autoclave, du fait de la non étanchéité desdits récipients.

Afin de résoudre ce problème de la conservation hors autoclave de la stérilité des récipients recevant les objets à traiter jusqu'au moment de l'utilisation de ceux-ci, on a imaginé d'enfermer les objets dans des poches souples en matériau en feuille spécial. Ces poches de stérilisation sont constituées, d'un côté, d'une feuille transparente imperméable et, de l'autre, d'une feuille perméable à l'air mais étanche aux germes, les deux feuilles étant scellées par thermosoudage après introduction des objets à stériliser.

Dans la pratique, il s'avère que l'étanchéité aux germes de la seconde feuille n'est que statistique et n'a donc rien d'absolu et qu'elle est de surcroît sérieusement obérée par les difficultés pratiques rencontrées lors de la fermeture des poches.

Une telle fermeture par thermocollage est, en effet, délicate et entraîne de nombreux rebuts qui accroissent les coûts de revient du fait du prix relativement élevé des poches; et, souvent, afin de compléter, par souci de sécurité, cette fermeture, les utilisateurs effectuent un agrafage qui malheureusement rend très aléatoire la conservation de la stérilité du contenu des poches après autoclavage. Et puis, de toutes manières, ces poches n'assurent pas mieux que les tambours de stérilisation, un bon séchage des objets.

Le but de la présente invention est précisément, dans le cadre d'une stérilisation à la vapeur en autoclave, de proposer un nouveau procédé assurant tout à la fois un excellent séchage des objets et une garantie totale de conservation de la stérilité après autoclavage jusqu'au moment de l'utilisation des objets quel que soit le lieu de cette utilisation.

A cet effet, l'invention a pour objet un procédé de stérilisation à la vapeur et de séchage d'objets solides caractérisé en ce qu'il consiste à enfermer les objets avec une quantité d'eau déminéralisée déterminée dans un récipient, à placer le récipient dans une enceinte isothermique, à admettre dans l'enceinte un fluide caloporteur à une température de l'ordre de 120° à 135°C environ en vue de vaporiser l'eau à l'intérieur du récipient, à laisser l'intérieur de l'enceinte à ladite température pendant un temps suffisant pour assurer la stérilisation, puis à extraire la vapeur d'eau contenue dans ce dernier en mettant en dépression l'intérieur du récipient et, enfin, à remettre l'intérieur du récipient à la pression atmosphérique ou au voisinage de celle-ci et à retirer le récipient de ladite enceinte.

Suivant un premier mode de mise en oeuvre de ce procédé on agence le récipient de manière à mettre en communication l'intérieur avec l'extérieur seulement lorsque la pression interne est supérieure à la pression externe, on met, à la fin du temps de stérilisation, l'intérieur de l'enceinte en dépression par rapport à l'intérieur du récipient pour extraire une fraction de la vapeur d'eau contenue dans ce dernier, on réinjecte dans l'enceinte du fluide caloporteur à ladite température en vue de revaporiser l'eau de condensation subsistant dans le récipient, on extrait à nouveau une partie de l'eau revaporisée dans le récipient en remettant en dépression l'intérieur de l'enceinte, on recommence le cycle réinjection de calories - extraction autant de fois que nécessaire pour avoir à l'intérieur du récipient une atmosphère de siccité désirée et, enfin, on met l'enceinte à l'atmosphère.

Un tel procédé permet de conserver la stérilisation absolue des objets contenus dans le récipient jusqu'à l'ouverture de ce dernier. En effet, une fois sorti de l'autoclave, le récipient conserve une étanchéité parfaite du fait que l'intérieur se trouve en forte dépression par rapport à l'atmosphère extérieure et que le récipient est ainsi conçu qu'aucune communication entre l'intérieur et l'extérieur ne peut s'opérer lorsque ⌐————⌐ la pression extérieure est supérieure à la pression intérieure.

L'invention concerne également un dispositif pour la mise en oeuvre du procédé ci-dessus caractérisé en ce qu'il est constitué d'un récipient comprenant un corps de récipient ouvert, un élément de fermeture de ce corps amovible, des organes d'étanchéification de la jonction entre le corps de récipient et l'élément de fermeture et un dispositif faisant office de soupape anti-retour assurant la communication avec l'extérieur uniquement dans le sens intérieur vers l'extérieur.

Suivant un second mode de mise en oeuvre du procédé de l'invention, on agence le récipient de manière à mettre en communication l'intérieur avec l'extérieur dans les deux sens lorsque la différence de pression atteint un seuil prédéterminé, on soumet, avant la phase de stérilisation proprement dite, l'intérieur de l'enceinte à une alternance de mise en pression - mise en dépression à l'aide dudit fluide caloporteur à ladite température afin d'extraire par fraction l'air contenu dans le récipient, puis, après stérilisation, on met l'enceinte en dépression et on réalise à partir de ce niveau de dépression des variations de dépression légèrement supérieures audit seuil prédéterminé afin d'obtenir un cycle alterné de mini-introductions de fluide caloporteur dans le récipient et de mini-extractions hors de ce dernier, puis, enfin, on remet la pression dans l'enceinte à l'atmosphère après passage par une valeur positive supérieure audit seuil prédéterminé, ce repassage à ladite valeur positive s'effectuant de préférence en substituant au fluide caloporteur un gaz stérile protecteur.

Ce second mode de mise en oeuvre permet notamment de maintenir dans le récipient jusqu'au moment de son ouverture sur le lieu d'utilisation, une pression résiduelle légèrement supérieure à l'atmosphère à l'aide d'un gaz tel que de l'azote stérile, filtré à 0,22 µ faisant office de barrière physique contre l'entrée des germes.

Suivant un troisième mode de mise en oeuvre, on agence le récipient de manière à connecter, une fois en place dans l'enceinte, l'intérieur directement avec l'extérieur de l'enceinte, sans communication avec l'intérieur de l'enceinte, puis on soumet l'intérieur du récipient, pendant

que l'enceinte est soumise à la vapeur d'eau, à une phase d'extraction de l'air contenu dans le récipient, de préférence par étapes en admettant directement dans le récipient de la vapeur d'eau et en procédant par alternances de pression-dépression, on stérilise à l'intérieur du récipient en maintenant le fluide caloporteur aux conditions de température, pression et temps appropriées, ensuite on sèche l'intérieur du récipient en mettant ledit intérieur du récipient en pression, de préférence à une pression légèrement supérieure à celle de l'atmosphère, à l'aide d'un gaz stérile protecteur, la pression dans l'enceinte étant ramenée à l'atmosphère à la fin de la période du séchage.

Ce mode de mise en oeuvre permet d'introduire dans le récipient,indépendamment de la nature et des conditions du fluide dans l'enceinte, divers fluides tels que de la vapeur d'eau saturée, des agents de lavage, brassage, siliconage, de l'azote de protection et de brancher directement une source de vide.

D'autres caractéristiques et avantages du procédé de l'invention ressortiront de la description qui va suivre des modes de mise en oeuvre ci-dessus, description donnée à titre d'exemple uniquement et en regard des dessins annexés sur lesquels :

- Fig. 1 représente schématiquement une vue en coupe longitudinale axiale d'un premier mode de réalisation d'un récipient propre à mettre en oeuvre le procédé de l'invention suivant un premier mode ;

- Fig. 2 représente une vue de droite du dispositif de la Fig. 1 ;

- Fig. 3 représente une coupe schématique suivant la ligne III - III du dispositif de la Fig. 1, et

- Fig. 4 représente en coupe schématique partielle un second mode de réalisation.

- Fig. 5 illustre un second mode de mise en oeuvre du procédé de l'invention ;

- Fig. 6 représente une coupe axiale d'un dispositif à double soupape pour la mise en oeuvre du procédé de l'invention suivant un troisième mode et,

- Fig. 7 illustre ce troisième mode de mise en oeuvre.

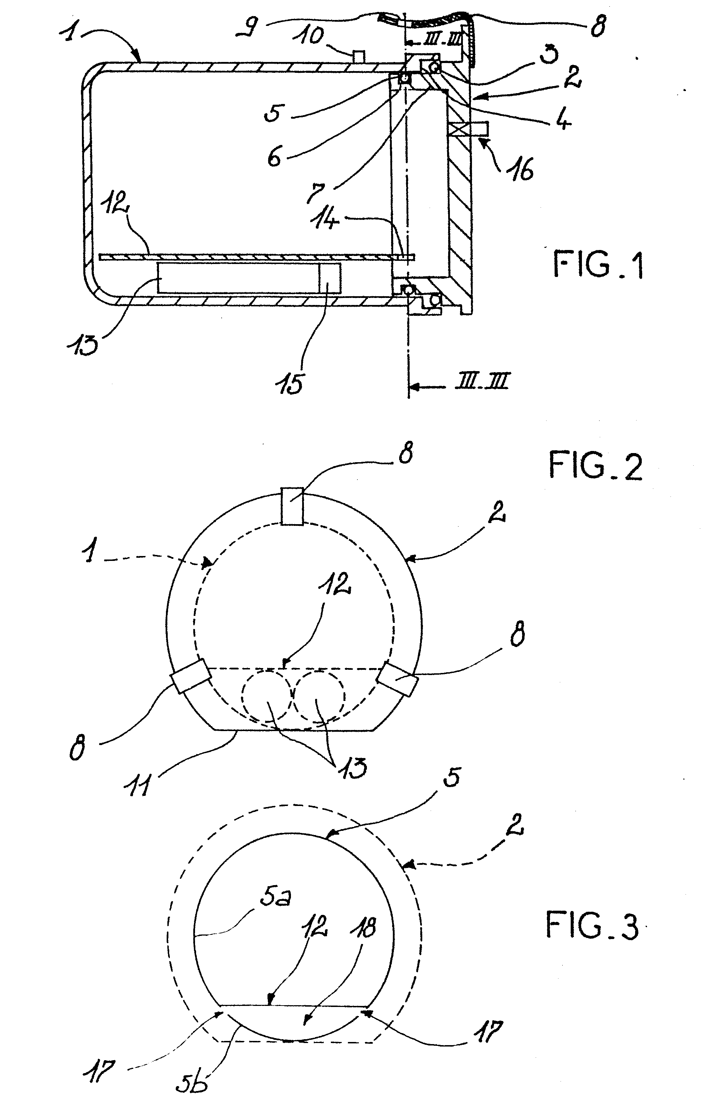

Le dispositif représenté sur les Fig. 1 à 3 est constitué d'un récipient comprenant un corps 1 cylindrique en matériau non oxydable et propre à supporter de fortes différences de pression de part et d'autre des parois et un couvercle 2 amovible susceptible de s'adapter de manière parfaitement étanche sur l'orifice du corps de récipient 1.

A cet effet, il est prévu un premier joint d'étanchéité torique 3 interposé entre le couvercle 2 et un épaulement 4 du rebord du corps de récipient 1 et un second joint d'étanchéité torique 5 en partie logée dans une gorge circulaire 6 réalisée sur la face externe d'un prolongement cylindrique interne 7 du couvercle 2. Ce second joint 5 coopère avec la face cylindrique interne du corps de récipient 1.

Le couvercle 2 est solidarisé du corps de récipient 1 à l'aide de trois (ou davantage) bandes 8 en matériau élastique à base de silicones capable de résister à la vapeur.

Une extrémité des bandes 8 est fixée sur le couvercle et l'autre extrémité est pourvue d'une boutonnière 9 permettant la fixation des bandes (en légère tension) sur des tétons d'accrochage 10 fixés sur le corps de récipient de manière à obturer le récipient, le joint 3 étant alors légèrement comprimé.

Le diamètre externe du couvercle 2 est nettement supérieur à celui du corps de récipient et comporte un méplat 11 en sorte que le récipient avec son couvercle puisse reposer allongé sans pouvoir rouler ni d'un côté ni de l'autre.

L'intérieur du récipient comporte une étagère ou caillebotis 12, plane et horizontale, parallèle au méplat 11 et reposant sur deux tubes à eau 13 couchés dans le bas du corps du récipient 1 parallèlement à son axe.

Afin que l'étagère amovible 12 soit bien positionnée dans le récipient, on prévoit dans le rebord du prolongement interne 7 du couvercle 2, deux encoches 14 de réception du rebord avant de l'étagère.

De la sorte, si l'étagère 12 n'est pas bien positionnée par rapport au méplat 11 du couvercle 2, ce dernier ne peut pas être complètement engagé et fixé au corps de récipient.

Les tubes 13 contiennent une certaine quantité prédéterminée d'eau déminéralisée et sont obturés par un bouchon 15 susceptible de sauter de lui-même sous l'effet de la vaporisation de l'eau.

Enfin, le couvercle est muni d'une soupape symbolisée en 16 et obturée par un bouchon, l'ensemble étant par exemple constitué par un robinet-pointeau et servant à la mise à l'atmosphère de l'intérieur du récipient au moment de l'ouverture.

Dans cette soupape 16 est inséré un filtre stérilisant à 0,22 µ.

La Fig. 3 illustre la conformation particulière du joint torique 5, lequel est interrompu en deux endroits symétriques 17.

Le joint 5 comprend donc une partie supérieure 5a dont les deux extrémités se situent légèrement, en position de stérilisation, en dessous du plan de l'étagère 12 et une partie inférieure 5 b dont les deux extrémités servent de trop-plein pour l'eau comme on le verra par la suite.

La stérilisation-séchage selon l'invention s'opère de la manière suivante.

Après mise en place des tubes 13 avec la quantité appropriée d'eau et de l'étagère 12 simplement posée sur les tubes, les objets à stériliser sont posés sur l'étagère et le récipient fermé par accrochage des bandes 8 sur les tétons 10.

Le récipient est alors placé en autoclave ou autre enceinte isothermique appropriée, le méplat 11 du couvercle reposant sur le plancher de l'autoclave.

De la vapeur (ou un autre fluide caloporteur) est envoyé dans l'enceinte, la température étant comprise entre 120° C et 135° C et la pression de l'ordre de 2 bars.

La pression à l'intérieur du récipient étant nettement inférieure à celle dans l'autoclave, l'étanchéité de la fermeture du récipient est parfaite.

Lors de la vaporisation de l'eau contenue dans les tubes 13, la montée en pression à l'intérieur de ces derniers provoquera à un certain moment l'expulsion (douce) dès bouchons 15, permettant à l'eau restante de se répandre dans le bas du récipient mais à un niveau sensiblement inférieur à celui de l'étagère 12.

Au bout d'un certain temps toute l'eau est vaporisée. Au cours de cette phase de vaporisation, la pression à l'intérieur du récipient devient supérieure à celle de l'intérieur de l'autoclave. Dès que ceci s'opère, le couvercle 2 tend à s'éloigner du corps de récipient 1 grâce à l'élasticité des bandes 8 et dès que cette course d'éloignement atteint 1 ou 2 mm le joint 3 ne fait plus obstacle à la sortie vers l'extérieur de l'air initial contenu dans le récipient. A la fin de la vaporisation, il ne subsiste alors plus pratiquement que de la vapeur d'eau saturée dans le récipient.

Le rôle de trop-plein du joint 5 b s'observe au cours de cette phase de décollement du couvercle. En effet, le liquide peut s'échapper également du récipient, le niveau de l'eau subsistante s'établissant (ligne 18, Fig. 3) à la hauteur des extrémités du segment de joint 5 b.

Le segment de joint supérieur 5a participe aussi, de par sa disposition, à une évacuation préférentielle et plus efficace de l'air initial sous la pression de la vapeur d'eau naissante à l'intérieur du récipient. En effet, cette vapeur naissante se porte de préférence à la partie supérieure du récipient, or le contenu gazeux du récipient ne peut s'échapper que par les deux passages 17 réalisés dans le joint 5, lesquels sont à la partie inférieure, au ras de l'eau.

La stérilisation des objets s'opère en vapeur saturée à 134 ° C environ et dure au moins 3 mn.

Il serait, bien entendu, possible de faire une extraction de l'air contenu dans le récipient avant envoi dans l'autoclave du fluide caloporteur à 120 -134°C. Il suffirait de mettre l'intérieur de l'autoclave en dépression pour aspirer l'air du récipient par la même "expansion" du couvercle 2 par rapport au corps de récipient 1 que celle décrite à l'instant.

Après quoi, on introduit le fluide caloporteur pour vaporiser l'eau à l'intérieur du récipient et obtenir une atmosphère de vapeur d'eau saturée à environ 134°C. Cette température de stérilisation d'ailleurs peut être abaissée à 120-125° C en vapeur d'eau saturée. On réalise une aussi bonne stérilisation en abîmant moins les objets notamment lorsqu'il s'agit de textiles. Toutefois, dans la pratique, on utilisera le plus souvent possible une température de l'ordre de 134°C car elle permet une meilleure revaporisation des condensats après stérilisation.

En effet, conformément à l'invention, aussitôt après stérilisation, on extrait, en mettant sous vide l'intérieur de l'autoclave, le fluide caloporteur (vapeur d'eau) et une partie de la vapeur d'eau contenue dans le récipient (et échappée de celui-ci par expansion du couvercle). Cette opération provoque la recondensation d'une partie de l'eau à l'intérieur du récipient car toute la vapeur d'eau ne peut être extraite.

C'est cette eau de condensation ou tout au moins une partie qui va être maintenant extraite par un cycle de revaporisation-extraction qui sera répété autant de fois que l'on veut afin d'aboutir au degré de siccité désiré à l'intérieur du récipient.

La revaporisation consiste à réinjecter du fluide caloporteur à 134° C préférentiellement dans l'autoclave. Cet apport de calories revaporise donc l'eau de condensation à l'intérieur du récipient.

Ensuite, on refait le vide dans l'autoclave pour enlever du récipient une nouvelle fraction de vapeur.

Il est à noter qu'au cours de ces diverses opérations, aucun germe susceptible de se trouver à l'intérieur de l'autoclave ne risque de s'introduire à l'intérieur du récipient car les seuls déplacements possibles de matière entre l'intérieur et l'extérieur du récipient n'ont lieu que dans le sens intérieur vers l'extérieur. Tant que la pression à l'intérieur de l'autoclave est sensiblement inférieure à celle à l'intérieur du récipient, il s'établit un flux de matière de l'intérieur du récipient vers l'extérieur. Mais dès que cette pression dans l'autoclave atteint un certain seuil (légèrement inférieur à la pression à l'intérieur du récipient en tenant compte des forces de rappel (bandes 8) du couvercle 2) l'étanchéité est rétablie et ne fait que se renforcer si la pression dans l'autoclave s'amplifie.

En fin de traitement, l'autoclave est mis à l'atmosphère. L'intérieur du récipient se trouve en dépression et parfaitement isolé de l'extérieur. Le récipient peut donc être sorti de l'autoclave, manipulé et transporté n'importe où sans risque de contamination des objets stérilisés et parfaitement secs contenus à l'intérieur. En particulier, le récipient peut passer dans des trappes germicides pour décontaminer la paroi externe.

La soupape 16 permet l'ouverture aisée du récipient au moment de l'utilisation des objets stérilisés. Il suffit d'enlever le bouchon recouvrant la soupape et manoeuvrer le robinet-pointeau de celle-ci pour la mise à l'air. Cette mise à l'air ne contamine pas l'intérieur du récipient grâce au filtre stérilisant incorporé à la soupape, lequel sera stérilisé à chaque nouvelle stérilisation.

Le couvercle 2 du récipient est avantageusement muni d'un moyen de vérification de la dépression interne. Si cette dépression n'est pas conforme à celle attendue c'est qu'il y a eu des problèmes en cours de stérilisation pouvant provenir d'une erreur de manipulation (manque d'eau par exemple), d'un joint d'étanchéité abîmé, etc...

Suivant un autre avantage du procédé de l'invention les objets à stériliser peuvent être introduits mouillés dans le récipient, ce qui supprime les opérations fastidieuses de séchage préalable dans la technique classique de stérilisation en autoclave.

On a vu plus haut que les encoches 14 ménagées dans le couvercle 2 constituaient un détrompeur permettant la vérification simple et instantanée de la bonne mise en place de l'étagère 12 et des tubes d'eau 13.

On peut aussi renforcer la sécurité en prévoyant un détrompeur pour la vérification qu'il y a bien de l'eau dans les tubes avant chaque nouvelle stérilisation.

L'absence d'eau dans le récipient sera de toute façon détectée en fin de stérilisation du fait que la valeur de la dépression à l'intérieur du récipient sera nettement différente de celle correspondant à une stérilisation normale à la vapeur.

Le récipient peut être également muni de hublots dans le couvercle et/ou le corps de récipient pour l'observation et le passage de la lumière ou bien une partie de sa paroi est en matériau transparent.

Le procédé de stérilisation de l'invention qui vient d'être décrit ci-dessus est le seul à présenter simultanément les avantages fondamentaux d'une stérilisation à température relativement réduite (vapeur à 120-135°C) et à l'état parfaitement sec et d'une conservation absolue de cette stérilité jusqu'au moment de l'utilisation sur place des objets traités.

Ce procédé se caractérise également par sa facilité de mise en oeuvre, par la souplesse d'emploi des récipients de stérilisation et par l'économie qu'il procure grâce à la réutilisation totale desdits récipients.

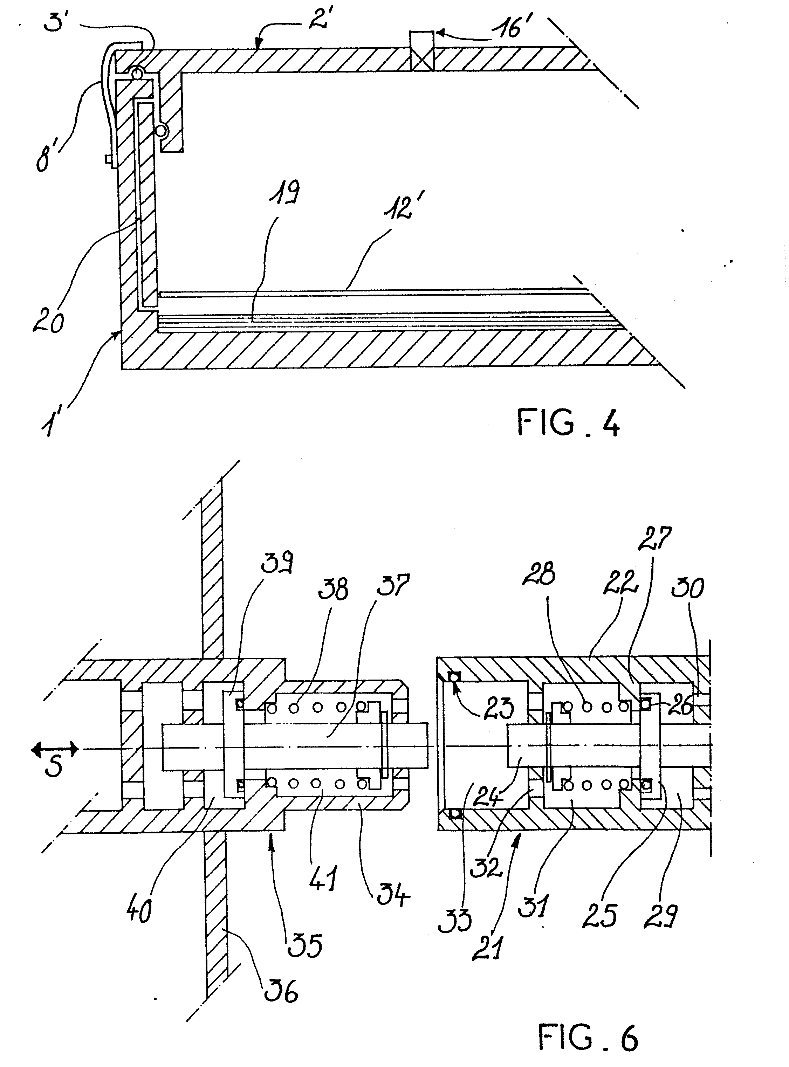

La Fig. 4 illustre une variante de réalisation du récipient de la Fig. 1 dans laquelle le corps de récipient 1'est parallèlèpipédique et fermé par un couvercle 2' tout-à-fait analogue à celui de la Fig. 1 avec ses joints 3' et 5', ses bandes de silicones 8' et sa soupape 16'.

L'eau (19) n'est plus contenue dans des tubes mais introduite directement dans le fond du récipient en dessous de l'étagère 12'.

Un canal 20 est ménagé dans la paroi du corps de récipient 1' et fait communiquer l'espace entre eau et étagère et l'espace entre joints 3',5'. Ces derniers sont des tores complets et le rôle du canal 20 équivaut à celui des interruptions 17 dans le joint 5, à savoir de privilégier l'extraction de l'air initial du récipient lors de la phase préliminaire de stérilisation consistant à évacuer l'air afin d'avoir une atmosphère de vapeur saturée dans le récipient.

Par ailleurs, le fonctionnement de ce récipient est tout-à-fait identique à celui qui vient d'être décrit à propos des Fig. 1 à 3.

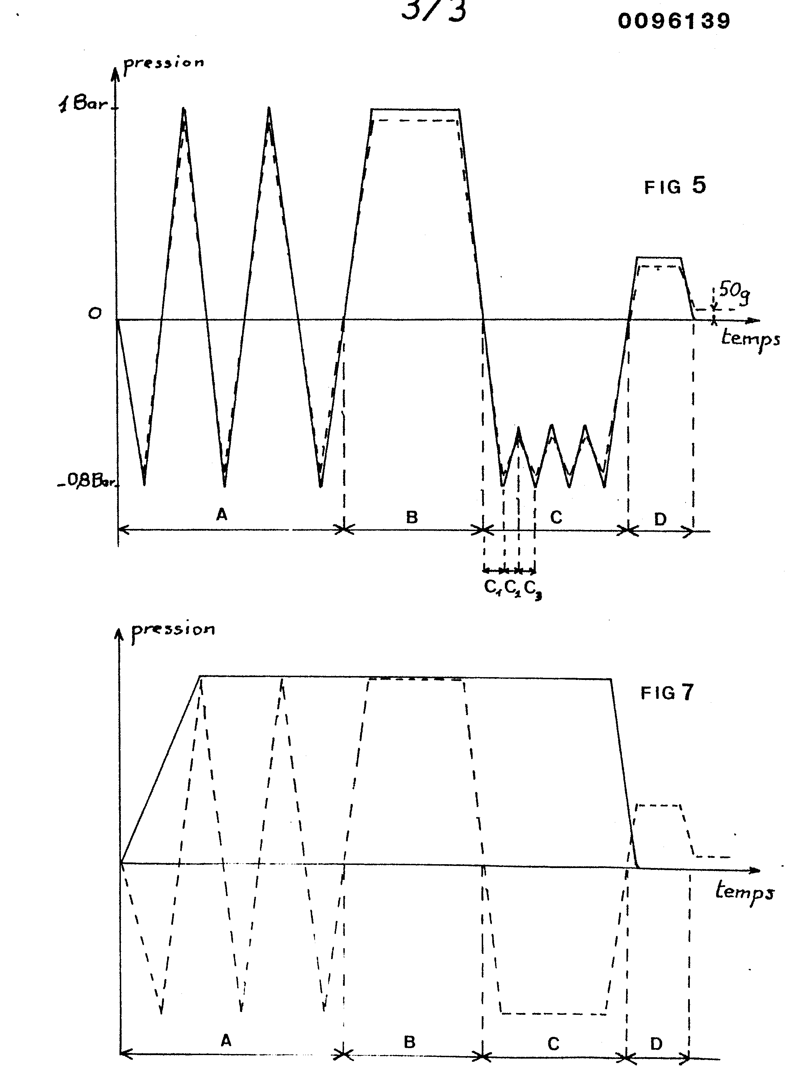

La fig. 5 illustre une variante du procédé selon laquelle on dispose entre l'intérieur du récipient et l'extérieur du récipient,et plus précisément l'intérieur de l'autoclave,un détendeur constitué par une valve ou soupape différentielle tarée par exemple à 50 g de pression et fonctionnant dans les deux sens. Ce moyen permet une introduction de vapeur à l'intérieur du récipient ainsi que le maintien à l'intérieur de ce dernier en fin de processus de stérilisation-séchage d'une surpression interne de 50 g environ par rapport à l'atmosphère, cette surpression obtenue grâce audit détendeur étant avantageusement réalisée à l'aide d'un gaz neutre tel que l'azote.

Sur la fig. 5 on a représenté en trait plein les variations de pression à l'intérieur d'un autoclave au cours d'un processus de stérilisation-séchage conforme à la variante ci-dessus. Les variations résultantes de pression à l'intérieur du récipient placé lui-même à l'intérieur de l'autoclave sont représentées en traits tiretés.

La phase initiale A est une phase de purge de l'air contenu dans le récipient. Celui-ci pouvant avoir une structure analogue à celles représentées sur les fig. 1 à 4 excepté pour ce qui concerne l'ensemble 8-9 remplacé par une soupape différentielle tarée à 50 g fonctionnant dans les deux sens et incorporée dans une paroi du récipient dont le couvercle est appliqué hermétiquement contre le corps du récipient par tous moyens appropriés.

Au cours de la phase A l'intérieur de l'autoclave est porté à plusieurs reprises alternativement en dépression (-0,8 bar par exemple) par rapport à l'atmosphère extérieure à l'autoclave, puis en surpression (1 bar par exemple). Dès que la différence de pression entre l'intérieur et l'extérieur du récipient dépasse 50 g en dépression comme en surpression, de la vapeur passe de l'intérieur de l'autoclave à l'intérieur du récipient ou bien une fraction de l'air contenu dans le récipient passe dans l'autoclave.

Cette vapeur (à 120-135° C) vaporise l'eau contenue dans le récipient comme on l'a vu plus haut. En fin de phase A il n'y a plus pratiquement que de la vapeur d'eau saturée à l'intérieur du récipient.

La phase B est la phase de stérilisation qui s'opère dans les mêmes conditions que plus haut.

La phase C est la phase de séchage au cours de laquelle on extrait l'eau de condensation formée à l'in-

- 13 -

0096139

térieur du récipient lors de la mise sous vide (phase C1) de l'autoclave. Cette extraction se fait par une alternance de mini-admissions de vapeur dans le récipient (introduction de calories-phases C2) et de mini-purges (mise sous vide différentiel - phases C3).

En fin de phase C (retour à la pression atmosphérique) toute l'eau du récipient est extraite.

Cette phase de séchage s'opère à une température par exemple de 134°C et pendant le temps nécessaire pour faire le nombre de mini-purges désiré.

Enfin, la phase C est immédiatement suivie d'une phase D au cours de laquelle de l'azote stérile filtré à 0,22 μ est introduit dans l'autoclave à une pression suffisante pour pénétrer dans le récipient.

Au retour à la pression atmosphérique dans l'autoclave (fin de la phase D) il subsiste dans le récipient une surpression de 50 g due à l'azote et qui formera une barrière physique contre l'entrée des germes.

Les fig. 6 et 7 illustrent encore une autre variante du procédé de l'invention consistant à raccorder l'intérieur du récipient à l'extérieur de l'autoclave une fois le récipient mis en place dans ce dernier.

La fig. 6 représente un mode de réalisation d'un tel raccordement.

On a désigné par 21 une soupape solidaire du récipient (non représenté) lequel peut être identique à ce qui est représenté sur les fig. 1 à 4 excepté en ce qui concerne l'ensemble 8-9 qui est supprimé, le couvercle étant fermement et hermétiquement appliqué par tous moyens appropriés contre le corps du récipient.

La soupape 21 comprend un manchon cylindrique 22 pourvu intérieurement au voisinage de son extrémité d'un joint torique 23.

A l'intérieur du manchon 22 est monté un poussoir axial 24 solidaire d'un clapet d'obturation 25 pourvu d'un joint torique 26 coopérant avec un épaulement circulaire 27 solidaire du manchon 22. Un ressort 28 repousse constamment le poussoir 24 de façon à appliquer le clapet 25 contre l'épaulement 27 et à isoler la chambre 29 (en communication avec l'intérieur du récipient par des passages 30) de la chambre 31 communiquant elle-même par des passages 32 avec une pré-chambre 33 susceptible de venir coiffer l'extrémité cylindrique 34 d'une soupape 35 montée dans la paroi 36 de l'autoclave.

Dans la soupape 35 est monté un poussoir axial 37 du même type que le poussoir 24 et comprenant un ressort de rappel 38 et un clapet étanche 39 séparant une chambre 40 susceptible d'être mise en communication avec une source S d'un fluide quelconque (vapeur d'eau, azote, etc..) ou de vide, d'une chambre 41 susceptible de communiquer avec la chambre 33.

En position de repos des clapets 25,39 comme représenté sur la fig. 6 le récipient et l'autoclave sont isolés de l'extérieur respectivement.

Lors de la mise en place du récipient dans l'autoclave la soupape 21 est embrochée sur la soupape 35.

Au cours de la première partie du mouvement d'embrochage, l'étanchéité de l'intérieur du manchon 22 est assurée par le joint 23 avant que les poussoirs 24-37 ne se soient repoussés mutuellement. Ce n'est qu'en fin d'embrochage que les deux clapets 25-39 s'écartent simultanément de leur siège respectif et ouvrent la communication entre l'intérieur du récipient et la source S.

Pour la déconnection il en sera de même , les communications étant interrompues avant que le joint 23 n'ait quitté le manchon 34. Ceci permet de ménager une sorte de sas de sécurité entre l'intérieur et l'extérieur du récipient tout en isolant l'intérieur de l'autoclave.

Avec cette variante de dispositif le processus de stérilisation-séchage s'opère comme illustré par la fig. 7 sur laquelle sont représentées en trait plein les variations de pression à l'intérieur de l'autoclave et en traits tiretés celles dans le récipient.

Pendant la phase A de purge de l'air du récipient, l'intérieur du récipient est soumis à une alternance de pression et dépression à l'aide de vapeur d'eau provenant de S et transitant par les deux soupapes 21-35, alors que l'intérieur de l'autoclave, indépendamment, est porté à une pression de vapeur d'eau constante (entre 120 et 135°C par exemple).

L'extraction de l'air du récipient se fait de manière pratiquement complète après plusieurs cycles d'admission-extraction comme dans le cas de la Fig. 5.

La phase A est suivie de la phase B de stérilisation (pression de vapeur d'eau saturée constante dans le récipient) puis de la phase de séchage C au cours de laquelle l'intérieur du récipient est mis en dépression (S étant alors une source de vide) à une valeur constante. Enfin, au cours de la dernière phase D la pression dans le récipient est rétablie à une valeur positive (par rapport à l'atmosphère) à l'aide de préférence d'une source S d'azote stérile et filtré à 0,22 μ afin de laisser subsister dans le récipient une pression résiduelle d'azote de quelques dizaines de grammes par rapport à l'atmosphère comme dans le cas de la Fig. 5.

La pression de l'autoclave est ramenée à zéro à la fin de la phase C. Il est à noter que cette pression dans l'autoclave est établie de façon à être très légèrement supérieure à la pression dans le récipient au cours de la phase B de stérilisation afin d'éviter un assèchement à l'intérieur du récipient.

L'avantage de l'isolement du récipient vis-à-vis de l'intérieur de l'autoclave est de permettre en plus sur les produits dans le récipient différents traitements tels que lavage ou siliconage par introduction directe des agents appropriés ou brassage par de l'air comprimé, par l'intermédiaire des deux soupapes en tandem 21-35.

Bien entendu, l'invention n'est pas limitée aux modes de réalisation représentés et décrits ci-dessus mais en couvre au contraire toutes les variantes, notamment en ce qui concerne le dispositif d'expansion du contenu du récipient vers l'extérieur faisant office de soupape anti-retour, lequel pourrait être constitué par tout autre dispositif ou agencement propre à assurer les mêmes effets techniques. Il en est de même pour le système à double soupape de la Fig. 6.

# REVENDICATIONS

:=:=:=:=:=:=:=:=:=:=:=:=:

1. Procédé de stérilisation à la vapeur et de séchage d'objets solides caractérisé en ce qu'il consiste à enfermer les objets avec une quantité d'eau déminéralisée déterminée dans un récipient, à placer le récipient dans une enceinte isothermique, à admettre dans l'enceinte un fluide caloporteur à une température de l'ordre de 120° à 135°C environ en vue de vaporiser l'eau à l'intérieur du récipient, à laisser l'intérieur de l'enceinte à ladite température pendant un temps suffisant pour assurer la stérilisation, puis à extraire la vapeur d'eau contenue dans ce dernier en mettant en dépression l'intérieur du récipient et, enfin, à remettre l'intérieur du récipient à la pression atmosphérique ou au voisinage de celle-ci et à retirer le récipient de ladite enceinte.

2. Procédé pour la mise en oeuvre suivant un premier mode du procédé de la revendication 1 caractérisé en ce qu'on agence le récipient de manière à mettre en communication l'intérieur avec l'extérieur seulement lorsque la pression interne est supérieure à la pression externe, on met, à la fin du temps de stérilisation, l'intérieur de l'enceinte en dépression par rapport à l'intérieur du récipient pour extraire une fraction de la vapeur d'eau contenue dans ce dernier, on réinjecte dans l'enceinte du fluide caloporteur à ladite température en vue de revaporiser l'eau de condensation subsistant dans le récipient, on extrait à nouveau une partie de l'eau revaporisée dans le récipient en remettant en dépression l'intérieur de l'enceinte, on recommence le cycle réinjection de calories-extraction autant de fois que nécessaire pour avoir à l'intérieur du récipient une atmosphère de siccité désirée et, enfin, on met l'enceinte à l'atmosphère.

3. Procédé suivant la revendication 2 caractérisé en ce que préalablement à l'admission dudit fluide caloporteur dans ladite enceinte, celle-ci est mise sous-vide afin d'extraire dudit récipient une partie de l'air initial enfermé.

- 2 -

0096139

4. Procédé pour la mise en oeuvre suivant un second mode du procédé de la revendication 1 caractérisé en ce qu'on agence le récipient de manière à mettre en communication l'intérieur avec l'extérieur dans les deux sens lorsque la différence de pression atteint un seuil prédéterminé, on soumet, avant la phase de stérilisation proprement dite, l'intérieur de l'enceinte à une alternance de mise en pression-mise en dépression à l'aide dudit fluide caloporteur à ladite température afin d'extraire par fraction l'air contenu dans le récipient, puis, après stérilisation, on met l'enceinte en dépression et on réalise à partir de ce niveau de dépression des variations de dépression légèrement supérieures audit seuil prédéterminé afin d'obtenir un cycle alterné de mini-introductions de fluide caloporteur dans le récipient et de mini-extractions hors de ce dernier, puis, enfin, on remet la pression dans l'enceinte à l'atmosphère après passage par une valeur positive supérieure audit seuil prédéterminé, ce repassage à ladite valeur positive s'effectuant de préférence en substituant au fluide caloporteur un gaz stérile protecteur.

5. Procédé pour la mise en oeuvre suivant un troisième mode du procédé de la revendication 1 caractérisé en ce qu'on agence le récipient de manière à connecter, une fois en place dans l'enceinte, l'intérieur directement avec l'extérieur de l'enceinte, sans communication avec l'intérieur de l'enceinte, puis on soumet l'intérieur du récipient, pendant que l'enceinte est soumise audit fluide caloporteur, à une phase d'extraction de l'air contenu dans le récipient, de préférence par étapes en admettant directement dans le récipient du fluide caloporteur et en procédant par alternances de pression-dépression, on stérilise l'intérieur du récipient en maintenant le fluide caloporteur aux conditions de température, pression et temps appropriées, ensuite on sèche l'intérieur du récipient en mettant ledit intérieur en dépression et, enfin, on remet l'intérieur du récipient en pression, de préférence à une pression légèrement supérieure à celle de l'atmosphère, à l'aide d'un gaz stérile protecteur, la pression dans l'enceinte étant ramenée à l'atmosphère à la fin de la période du séchage.

6. Procédé suivant la revendication 5 caractérisé en ce qu'indépendamment de la nature et des conditions du fluide dans l'enceinte on introduit, à un moment ou à un autre du processus de stérilisation-séchage, divers agents ou fluides auxiliaires pour effectuer, par exemple, un lavage, brassage ou siliconage des produits.

7. Dispositif de stérilisation pour la mise en oeuvre du procédé selon la revendication 2 ou 3, caractérisé en ce qu'il est constitué d'un récipient comprenant un corps de récipient ouvert (1), un élément de fermeture (2) de ce corps amovible, des organes d'étanchéification (3,5) de la jonction entre le corps de récipient et l'élément de fermeture et un dispositif (8) faisant office de soupape anti-retour assurant la communication avec l'extérieur uniquement dans le sens intérieur vers l'extérieur.

8. Dispositif de stérilisation suivant la revendication 7 caractérisé en ce que le dispositif faisant office de soupape anti-retour est constitué par un organe (8) de liaison amovible du couvercle (2) au corps de récipient (1) en matériau approprié présentant une certaine élasticité, fixé à l'une des deux parties du récipient, le corps (1) ou le couvercle (2), et des moyens (10) d'accrochage dudit organe de liaison en position de fermeture étanche du couvercle (2), solidaires de l'autre partie du récipient.

9. Dispositif de stérilisation suivant la revendication 8 caractérisé en ce que ledit organe de liaison est constitué par une ou plusieurs bandes (8) mises en légère tension lors de l'accrochage de l'élément de fermeture du couvercle (2).

10. Dispositif de stérilisation suivant la revendication 8 ou 9 caractérisé en ce que le matériau dudit organe de liaison est du caoutchouc siliconé.

11. Dispositif de stérilisation pour la mise en oeuvre du procédé selon la revendication 4 caractérisé en ce qu'il comporte un corps de récipient ouvert, un élément de fermeture étanche de ce corps amovible, des moyens de verrouillage de cet élément de fermeture, et une soupape différentielle tarée permettant les échanges de l'intérieur vers l'extérieur et inversement au-delà d'un seuil de différence de pression déterminé, ladite soupape étant incorporée dans une paroi du dispositif.

12. Dispositif de stérilisation pour la mise en oeuvre du procédé suivant la revendication 5 caractérisé en ce qu'il comprend un corps de récipient, un organe de fermeture amovible et des moyens de mise en communication de l'intérieur du récipient avec l'extérieur de l'enceinte de stérilisation, lesdits moyens étant constitués d'une première soupape (21) incorporée dans une paroi du récipient et d'une seconde soupape (35) incorporée dans une paroi (36) de l'enceinte, les deux soupapes étant emboîtables de manière à assurer la liaison intérieur du récipient-extérieur de l'enceinte de manière étanche vis à vis de l'intérieur de l'enceinte.

13. Dispositif de stérilisation suivant l'une des revendications 7 à 12 caractérisé en ce qu'il comporte une étagère (12) destinée à supporter les objets à stériliser et au-dessous de laquelle est constituée une réserve d'eau.

14. Dispositif de stérilisation suivant la revendication 13, caractérisé en ce que la réserve d'eau est enfermée dans un ou plusieurs tubes (13) fermés par un bouchon (15) susceptible de libérer l'eau contenue lors de la vaporisation de celle-ci.

15. Dispositif de stérilisation suivant l'une des revendications 7 à 14 caractérisé en ce que l'étanchéité entre corps de récipient (1) et couvercle (2) est obtenue par un premier joint torique (3) interposé entre le couvercle et un épaulement frontal (4) ménagé dans un rebord circulaire du corps de récipient et un second joint torique (5) porté par le couvercle et coopérant avec la paroi cylindrique interne du corps de récipient.

16. Dispositif de stérilisation suivant la revendication 15, caractérisé en ce que le corps de récipient (1) est un cylindre fermé à une extrémité et ouvert à l'autre, l'étagère (12) support des objets à traiter est placée parallèlement à l'axe du récipient et le couvercle (2) ferme l'extrémité ouverte dudit cylindre.

17. Dispositif de stérilisation suivant la revendication 16, caractérisé en ce que le couvercle (2) présente un diamètre notablement supérieur à celui du corps de récipient (1) et présente un méplat ou pan coupé (11) parallèle au plan de l'étagère (12) et destiné à stabiliser latéralement le récipient.

18. Dispositif de stérilisation suivant l'une des revendications 15 à 17 caractérisé en ce que le couvercle (2) comporte un prolongement intérieur cylindrique portant lesdits joints toriques (3,5) et muni d'encoches (14) d'encastrement en position correcte de l'extrémité de ladite étagère (12).

19. Dispositif de stérilisation suivant l'une des revendications 15 à 18, caractérisé en ce que le second joint d'étanchéité (5) comprend deux segments distincts (5a, 5b) séparés par deux intervalles (17) situés en-dessous du plan de l'étagère (12).

20. Dispositif de stérilisation suivant la revendication 15, caractérisé en ce que le corps de récipient est parallèlèpipédique, l'étagère (12') reposant sur le fond parallèlement au plan du couvercle (2') et en ce qu'un canal (20) ménagé dans la paroi du corps de récipient (1') fait communiquer l'espace en-dessous de l'étagère (12') et l'espace délimité entre les deux joints d'étanchéité (3', 5') en position de fermeture du récipient.

21. Dispositif de stérilisation suivant l'une des revendications 7 à 20, caractérisé en ce qu'il comporte dans la paroi du corps du récipient (1, 1') ou du couvercle (2,2') une soupape de mise à l'air (16, 16').

22. Dispositif de stérilisation suivant l'une des revendications 7 à 21 caractérisé en ce qu'il comporte un dispositif de contrôle de la dépression interne du récipient.

23. Dispositif de stérilisation suivant l'une des revendications 7 à 22 caractérisé en ce qu'il comporte au moins un hublot ou partie de paroi transparente.

FIG.1

FIG.2

FIG.3

0096139

FIG . 4

FIG . 6

0096139

FIG 5

pression

1 Bar

O

-0.8 Bar

50 g

temps

A B C D

$C_1 C_2 C_3$

FIG 7

pression

temps

A B C D

# RAPPORT DE RECHERCHE EUROPEENNE

Office européen
des brevets

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. ³) |
|---|---|---|---|
| | --- | | A 61 L 2/06 |
| A | FR-A-2 335 239 (SANDERSON) *Revendications* | 1 | |
| | ----- | | |

DOMAINES TECHNIQUES RECHERCHES (Int. Cl. ³)

A 61 L

Le présent rapport de recherche a été établi pour toutes les revendications

| Lieu de la recherche | Date d'achèvement de la recherche | Examinateur |
|---|---|---|
| LA HAYE | 08-02-1983 | MALHERBE Y.J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82